# EUROPEAN PATENT SPECIFICATION

(11) **EP 0 565 604 B1**
(45) Date of publication and mention of the grant of the patent: **28.07.1999**
(21) Application number: 92903283.7
(22) Date of filing: 27.12.1991
(51) Int. Cl.: A61M 31/00, A61M 29/00, A61M 29/02

(54) **CATHETER FOR DRUG DELIVERY SYSTEM**
KATHETER ALS ARZNEIABGABESYSTEM
CATHETER COMPRENANT UN SYSTEME D'APPORT DE MEDICAMENT

(30) Priority: 28.12.1990 US 635732; 22.11.1991 US 795976
(43) Date of publication of application: 20.10.1993
(60) Divisional application: 99101203.0
(73) Proprietor: BOSTON SCIENTIFIC CORPORATION, Natick, MA 01760-1537 (US)
(72) Inventor: SAHATJIAN, Ronald, Lexington, MA 02173 (US)
(74) Representative: Deans, Michael John Percy
(86) International application number: US9109805
(87) International publication number: WO9211896

(56) References cited:
- EP-A- 0 372 088
- WO-A-89/12478
- WO-A-91/08790
- DE-C- 380 205
- US-A- 4 515 593
- US-A- 4 769 013
- US-A- 4 950 256
- US-A- 5 026 607
- US-A- 5 041 100

## Description

### Field of the Invention

The invention relates to delivery of drugs to the walls of body lumens.

### Background of the Invention

Systemic administration of drugs treats the organism as a whole, even though the disease may be localized, such as occlusion of a duct or vessel. Localization of a drug poses special problems in cases involving the walls of ducts and vessels, since, by nature, these organs serve as transport systems.

Atherosclerotic disease, for example, causes localized occlusion of the blood vessels resulting from the build-up of plaque. As the deposits increase in size, they reduce the diameter of the arteries and impede blood circulation. Angioplasty, which involves the insertion of catheters, such as balloon catheters, through the occluded region of the blood vessel in order to expand it, has been used to treat atherosclerosis.

The aftermath of angioplasty in many cases is problematic, due to restenosis, or closing of the vessel, that can occur from causes including mechanical abrasion and the proliferation of smooth muscle cells stimulated by the angioplasty treatment. Restenosis may also occur as a result of clot formation following angioplasty, due to injury to the vessel wall which triggers the natural clot-forming reactions of the blood.

WO 89/12478 discloses a dilatation catheter which has a dilatable balloon at one end which contains an active substance. The balloon wall may have a number of pore-like holes extending through it to allow the active substance inside the balloon to pass through when the balloon expands. In addition, the outer surface of the balloon may also carry a porous, spongy layer containing an active substance which is at least partly squeezed out when the layer presses against a blood-vessel wall. The balloon may also carry on its surface an annular layer which contains an active substance and which can be detached and can adhere to the inside wall of a blood vessel.

EP-A-0372088 discloses a balloon for a catheter in which all or part of the balloon membrane is composed of a drug-supporting material capable of gradually discharging a drug.

US 4905256 discloses an intravascular catheter comprising a cannula for insertion into a vascular system of a patient which is coated with a hydrophilic polymer containing, in the polymer, an effective amount of an antimicrobial anticoagulant consisting essentially of polymyxin to prevent the growth of microorganisms and the formation of blood clots on the coating.

### Summary of the Invention

In one aspect, the invention features a catheter for delivering drug to tissue at a desired location of the wall of a body lumen. The catheter is constructed for insertion in a body lumen and has a catheter shaft and an expandable portion mounted on the catheter shaft. The expandable portion is expandable to a controlled pressure to fill the cross-section of the body lumen and press against the wall of the body lumen. At least a portion of the exterior surface of the expandable portion is defined by a coating of a tenaciously adhered swellable hydrogel polymer. Incorporated in the hydrogel polymer is an aqueous solution of a preselected drug to be delivered to the tissue. The hydrogel polymer coating has the characteristic of releasing the aqueous drug solution in response to compression of the coating against the wall of the lumen when the expandable portion is expanded.

Various embodiments may include one or more of the following features. The catheter is adapted for insertion in a blood vessel, and the expandable portion is an inflatable dilatation balloon adapted for inflation at pressures in the range for effecting widening of a stenosed blood vessel. The pressure is in the range of about 101.325 to 2026.500 kPa (about 1 to 20 atmospheres). The hydrogel polymer and drug are effective to release about 20% or more of the drug during inflation in the pressure range. The compression is effective to deliver the drug over a duration of about 10 minutes or less. The hydrogel polymer coating is about 10 to 50 microns thick in the swelled, uncompressed state. The hydrogel polymer is selected from the group consisting of polycarboxylic acids, cellulosic polymers, gelatin, polyvinylpyrrolidone, maleic anhydride polymers, polyamides, polyvinyl alcohols, and polyethylene oxides. The preferred hydrogel polymer is polyacrylic acid. The drug is an anti-thrombogenic drug selected from the group consisting of heparin, Pebac, enoxaprin, aspirin and hirudin. The drug is an anti-proliferative drug selected from the group consisting of monoclonal antibodies, capable of blocking smooth muscle cell proliferation, heparin, angiopeptin and enoxaprin. The expandable portion is adapted for application of heat to the polymer material to control the rate of administration. The catheter further comprises a sheath member, extendable over the balloon to inhibit release of the drug into body fluids during placement of the catheter. The balloon catheter is a perfusion catheter having an expandable balloon. The expandable portion includes a stent, mountable in the blood vessel by expansion thereof. The drug is bound in the hydrogel polymer for slow time release of the drug after the compression of the hydrogel polymer by the expansion. The hydrogel polymer is a polyacrylic acid including an ammonium cation and the drug is heparin. The stent is expandable by a balloon. The catheter where the stent and balloon both include the swellable hydrogel coating incorporating the drug. The expandable portion is prepared by introducing an aqueous solution of the drug to the hydrogel polymer coating, the catheter is introduced to the body lumen to position the expandable portion at the point of desired drug application, and the expandable portion is expanded to enable delivery of the drug by compression of the hydrogel polymer coating against the wall at the body lumen. The expandable portion is positioned at a point of occlusion in the blood vessel and expanding the expandable portion at pressures sufficient to simultaneously dilate the vessel and deliver the drug by compression of the hydrogel polymer coating.

In a particular aspect, the invention includes a balloon catheter for delivering drug to tissue at a desired location of the wall of a blood vessel. The catheter is constructed for insertion in a blood vessel and has a catheter shaft and an expandable dilatation balloon mounted on the catheter shaft. The expandable balloon is expandable by an expansion controller to engage the tissue at a controlled pressure in the range of about 101.325 to 2026.500 kPA (about 1 to 20 atmospheres) to fill the cross-section of the blood vessel and press against the wall of the blood vessel. At least a portion of the exterior surface of the expandable balloon is defined by a coating of a tenaciously adhered swellable hydrogel polymer with a thickness in the range of about 10 to 50 microns in the swelled state, and incorporated within the hydrogel polymer coating, an aqueous solution of a preselected drug to be delivered to the tissue.

In various embodiments, the hydrogel polymer is selected from the group consisting of polycarboxylic acids, cellulosic polymers, gelatin, polyvinylpyrrolidone, maleic anhydride polymers, polyamides, polyvinyl alcohols, and polyethylene oxides. The preferred hydrogel polymer is polyacrylic acid. The drug is an anti-thrombogenic drug selected from the group consisting of heparin, Pebac, enoxaprln, aspirin and hirudin. The drug is an anti-proliferative drug selected from the group consisting of monoclonal antibodies, capable of blocking smooth muscle cell proliferation, heparin, angiopeptin and enoxaprin. The catheter further comprises a sheath member, extendable over the balloon to inhibit release of the drug into body fluids during placement of the catheter.

In another aspect, the invention features a method for the manufacture of a catheter for delivering drug to tissue at a desired location of the wall of a body lumen, the catheter being constructed for insertion in a body lumen and having a catheter shaft and an expandable portion mounted on the catheter shaft, the expandable portion being expandable to a controlled pressure to fill the cross-section of the body lumen and press against the wall of the body lumen. In this method, at least a portion of the exterior surface of the expandable portion is coated with a tenaciously adhering swellable hydrogel polymer, and the drug to be delivered to the tissue is incorporated into the hydrogel polymer by introduction thereto as an aqueous solution. The hydrogel polymer coating has the characteristic of releasing said aqueous drug solution in response to compression of the coating against the wall of the lumen when the expandable portion is expanded.

In general, an advantage of the invention is the application of drugs by active diffusion directly into the tissue within the body requiring treatment. The drug is preferably applied in a rapid but low-stress, low energy manner that does not further injure the tissue to be treated, and administration is selectively and evenly distributed over the treated area such that the drug can be taken up by tissue and plaque, without, e.g., being washed away by body fluids.

### Description of Preferred Embodiments

We first briefly describe the drawings.

### Drawings

Figs. 1-1a are enlarged views of a method of preparing an embodiment of the invention.
Fig. 1b is an enlarged cross-sectional view of an embodiment of the drug delivery balloon catheter of the invention being moved through a vessel toward an occlusion to be treated.
Fig. 1c is an enlarged cross-sectional view of the balloon in Fig. 1b, now fully inflated and at the site of occlusion.
Fig. 1d is a further enlarged, schematic cross-sectional view of the portion of Fig. 1c indicated in the circle 1e, but taken prior to full inflation.
Fig. 1e, which corresponds to the portion of Fig. 1c indicated in the circle 1e, is an enlarged, schematic cross-sectional view, as in Fig. 1d, but with the balloon under full inflation to release the drug coated on the balloon.
Fig. 2 is an enlarged cross-sectional view of another embodiment of the drug delivery balloon catheter of the invention including a sheath for covering the catheter as it is being moved through a vessel toward the occlusion to be treated.
Fig. 2a is an enlarged cross-sectional view of the catheter of Fig. 2 with the sheath retracted and balloon inflated at the site of occlusion.
Fig. 3 is an enlarged, schematic cross-sectional view of another embodiment of the drug delivery balloon catheter in which the drug, originally held within a polymer applied to a thermal balloon of the invention, is now entering the surrounding tissue.
Fig. 3a is a further enlarged, schematic illustration of the embodiment of Fig. 3 and illustrates the entry of the drug, shown as circles, into the surrounding tissue.
Figure 4 shows a balloon catheter with the hydrogel and drug coated endoprosthesis mounted on the balloon section, in the region of the thrombus, before radial expansion of the balloon section and endoprosthesis.
Figure 4a is an enlargement of Figure 4 showing the hydrogel polymer and drug coated endoprosthesis and Fig. 4b is a cross-section along the line b-b in Fig. 4a.
Figure 5 shows the endoprosthesis compressed against the vessel wall by radial expansion of the balloon section with the drug diffused into the compressed thrombus before removal of the balloon catheter.
Figure 6 shows the endoprosthesis positioned against the drug inside the compressed thrombus, after removal of the balloon catheter.

### General Description

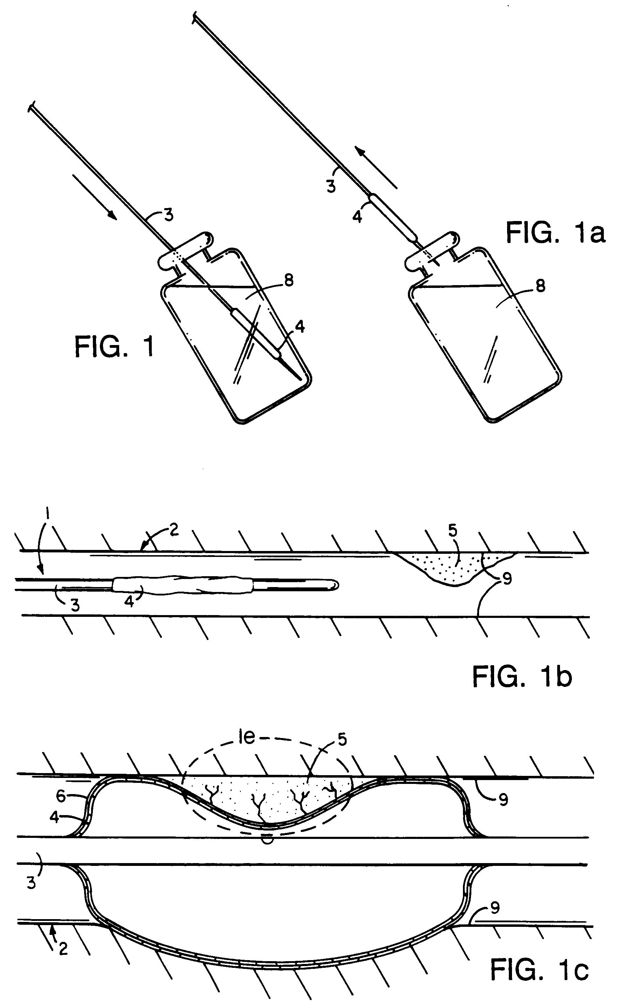

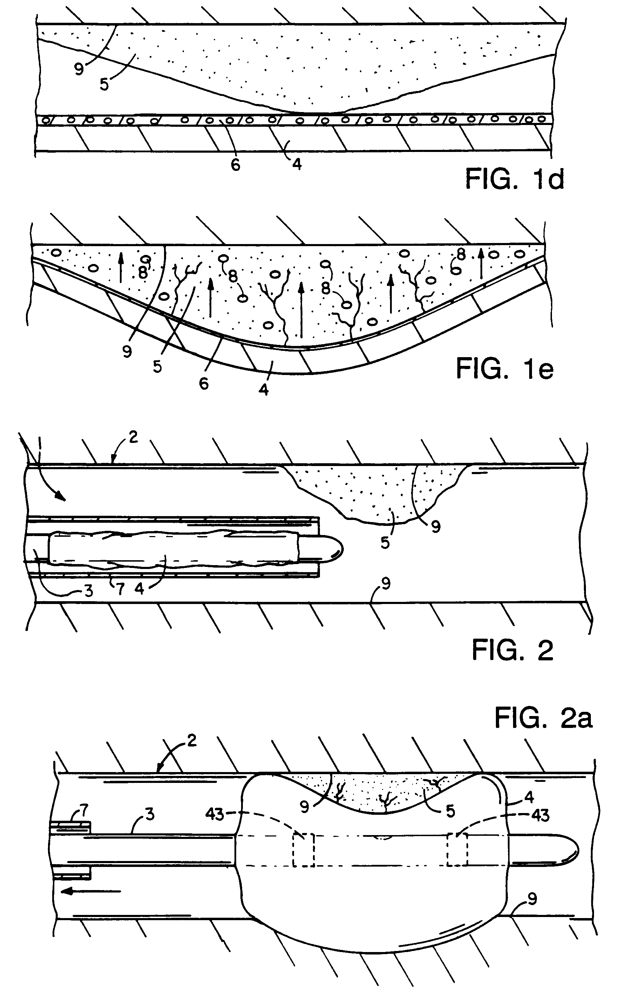

Referring to Figs. 1-1e, in one embodiment, the invention includes a drug delivery balloon catheter device 1 comprising a catheter body 3 having a balloon 4 attached at its distal end. The balloon 4 on the catheter 3 includes a swellable hydrogel polymer coating 6. As shown in Figs. 1-1a, a drug 8 in an aqueous solution is absorbed into the hydrogel with the balloon in the deflated state prior to insertion into the patient by the physician, e.g., the hydrogel-coated balloon may be immersed in a small tube or vial containing the drug. The drug may also be applied in the form of droplets, such as from an eyedropper, or the drug may be precipitated into the hydrogel prior to sterilization and sold as a finished device. Exposure of the hydrogel to the solution causes the hydrogel to swell.

As shown in Fig. 1b, typically the device 1 is inserted into the duct or vessel 2 having a region to be treated, such as an occlusion due to a deposition of plaque 5 on the vessel wall tissue 9. The device 1 is moved along the vessel to position the balloon 4 at the occlusion site, as shown in Fig. 1c. The vessel may be, for example, a narrow, tortuous opening through which the catheter is passed by torquing from the distal end. As the balloon is inflated the pressure created by the balloon against the tissue compresses the hydrogel and the drug is quickly and freely released for transfer by active diffusion into the plaque and tissue. The pressure applied to the plaque and tissue by the expansion of the balloon during application of the drug enhances transfer of the drug into the tissue and plaque. This process is referred to here as active diffusion. The balloon and catheter may be exposed to the body fluids of the lumen for a considerable time, e.g., up to about 15 minutes in some angioplasty procedures. An advantage of this invention is that large amounts of the drug, e.g., greater than 20%, even 30-50% or more, of the drug solution contained in the hydrogel, is diffused into the effected area in the short time duration which the hydrogel is compressed, e.g., 2-10 minutes after the balloon is inflated at the treatment site. The inflation pressure needed to dilate the vessel which also approximates the compression of the coating, is in the range of 101.325 to 2026.500 kPa (1 to 20 atmospheres), typically about 202.650 to 1013.250 kPa (about 2 to 10 atmospheres), The balloon is preferably a compliant material such as polyethylene which conforms to the shape of the lumen wall. The balloon may also be formed of other materials used in angioplasty, e.g., a nondistending material, such as polyethylene terephthalate (PET). Transporting the drug in the hydrogel prevents substantial release of the drug to body fluids prior to reaching the treatment area and during the drug application phase and allows large dosages to be delivered at a desired location.

In the embodiment of Fig. 1c, the balloon coating 6 is a swellable, compressible coating formed of the hydrogel and drug in solution. In Fig. 1d, the balloon 4 is shown inflated such that the coating 6, which has an initial thickness, is in contact with the occlusion 5 but not under substantial pressure. Further inflation of the balloon 4, as shown in Fig. 1e, compresses the hydrogel coating 6 against the occluded areas 5 causing quick release of the drug (represented by circles) contained in the coating 6 directly into the plaque and nearby healthy tissue, as indicated by the directional arrows, much in the nature of squeezing liquid from a sponge. The introduction of the drug into the plaque and tissue occurs simultaneously with widening of the occlusion by the dilatation balloon. Thus, as cracking of the plaque and stimulation of smooth muscle cells beneath the plaque and along healthy tissue of the vessel wall are caused by dilatation, a therapeutic drug is simultaneously applied to the effected area, e.g., to counteract the effects of the trauma. The thickness of the balloon 4 remains substantially the same, while the thickness of the coating 6 decreases due to the compression of the coating and the release of the drug 8. (Figs. 1d-1e are schematic drawings and are not to scale with respect to the thickness of the balloon relative to the thickness of the hydrogel coating.) The drug carried by the balloon is evenly applied to plaque and tissue and isolated by the pressure of the balloon from the flow of body fluids in the lumen such that the drug, e.g., an anti-proliferative, may actively diffuse through the cracks formed in the plaque and reach the smooth muscle tissue. (It will also be understood that, as an alternative procedure, after dilation with a conventional angioplasty balloon catheter, a catheter carrying a drug-delivery, inflatable balloon, such as has been described, may be used to treat the vessel.)

The hydrogel coating is characterized by the ability to incorporate a substantial amount of the drug, typically in aqueous solution form, and is swellable such that the aqueous drug solution can be effectively squeezed out of the coating when pressure is applied by inflation of the balloon. Administration of the drug in this way enables the drug to be site specific, such that release of high concentrations and/or highly potent drugs may be limited to direct application to the diseased tissue. Furthermore, the drug is applied to the diseased tissue by the sponge-like coating in an even, gentle manner without disrupting or injuring the healthy tissue, while diffusion of the drug into the tissue is facilitated by the application of the pressure of the inflated balloon. The pressure also effectively forms a seal that prevents the flow of body fluids from washing the drug downstream of the treatment area. The dosage applied to the tissue may be controlled by regulating the time of presoaking the drug into the hydrogel coating to determine the amount of absorption of the drug solution by the hydrogel coating. Other factors affecting the dosage are the concentration of the drug in the solution applied to the coating and the releasability of the hydrogel coating, determined by, for example, the thickness of the hydrogel coating, its resiliency, porosity and the ability of the hydrogel coating to retain the drug, e.g., electrostatic binding or pore size, or the ionic strength of the coating, e.g., changed by changing the pH.

The drug may be an anti-thrombogenic drug, such as heparin or a heparin derivative, Pebac (dextrophenylalanine proline arginine chloromethylketone) or an anti-proliferative, such as heparin (also known to have anti-proliferative properties), enoxaprin, angiopeptin, or monoclonal antibodies capable of blocking smooth muscle cell proliferation, or it may be hirudin or acetylsalicylic acid (i.e., aspirin). Dosages applied to the tissue, for example, of heparin are typically in the range of 10-30 mg of heparin solution containing 200-1,000 units of sodium heparin. For use with hydrogels, the drug is preferably water soluble, so that the drug may be easily absorbed into the coating matrix.

The sponge-like characteristics of the hydrogel allows the aqueous drug solution to be effectively squeezed out of the coating when pressure is applied by inflation of the balloon. The hydrogel and drug combination are preferably noncomplexed, i.e., held together through the ability of the hydrogel to swell and absorb the drug solution, thereby allowing the preferable free-release of the drug at the treatment site.

In particular embodiments it may be advantageous to select a hydrogel coating for a particular drug such that the drug is not substantially released into body fluids prior to application of pressure by expansion of the balloon. Binding of the drug may also be accomplished by electrostatic attraction of the drug to the coating or a coating additive or by mechanical binding, e.g., employing a coating having a pore size that inhibits inward flow of body fluids or outward flow of the drug itself, that might tend to release the drug. Hydrogels are particularly advantageous in that the drug is held within the hydrogen-bond matrix formed by the gel.

The hydrogel is a cross-linked polymer material formed from the combination of a colloid and water. Cross-linking reduces solubility and produces a jelly-like polymer that is characterized by the ability to swell and absorb a substantial amount of the drug, typically in aqueous solution form. The hydrogel coating is also particularly hydrophilic, water swellable, and lubricous (i.e., having a low coefficient of friction). Preferred hydrogels are polyacrylic acid polymers available as HYDROPLUS® (Boston Scientific, Watertown, MA) and as described in U.S. Patent No. 5091205, US application no. 297,331 filed January 17, 1991). The drug e.g., heparin in aqueous solution, is absorbed into the coating without complexing and is freely released therefrom. Such hydrogel-drug combinations deliver about half of the drug solution in response to pressures in the range of balloon angioplasty in the vascular system. In other particular embodiments, the hydrogel polymer includes acid groups and incorporates a drug which is anionic in nature that is bound by electrostatic attraction to cations in the coating, such as an ammonium cation, as described in "Lubricous Antithrombogenic Catheters, Guidewires, and Coatings," by Ronald Sahatjian et al., US Patent No. 5135516, USSN 7/451,507 filed on December 15, 1989 - (see also corresponding International Publication No. WO91/08790, published June 27, 1991). The coating incorporating the quaternary ammonium salt is effective to deliver an initial fast release of drug during compression and a slow release of drug remaining in the compressed coating after compression and is particularly useful for coating vascular stents as described further below.

In general, when dry, the hydrogel coating is preferably on the order of about 1 to 10 microns thick, with a 2 to 5 micron coating typical. Very thin hydrogel coatings, e.g., of about .2-.3 microns (dry) and much thicker hydrogel coatings, e.g., more than 10 microns (dry), are also possible. Typically, the hydrogel coating thickness may swell by about a factor of 6 to 10 or more when the hydrogel coating is hydrated. For example, a hydrogel coating of about 1 to 3 microns thickness, when dry, usually swells to about 10-30 microns thickness, when hydrated. Most preferably, the thickness of the coating is about 10 to 50 microns in the swelled, uncompressed state, and incorporates about 20-30mg of drug solution.

Referring to Fig. 2, in another embodiment of a drug delivery balloon catheter, the catheter 3 is preferably very small in diameter and flexible, much in the nature of a guidewire, formed, in this case, of hollow tubing to which the coated balloon 4 is attached. The balloon is covered by a protective sheath 7 while the instrument 1 is inserted into the vessel or duct 2 and positioned at the treatment region. As the coated balloon 4 is positioned at the occluded site 5, (Fig. 2a) the protective sheath 7 is drawn back to expose the balloon 4. In an alternative embodiment, the sheath remains stationary while the guidewire-like catheter moves the coated balloon forward into the occluded region. The sheath 7 protects the coating and inhibits premature release of the drug. Such a sheath might be particularly advantageous with coatings and drugs without substantial chemical or mechanical binding. Additionally, the balloon catheter may be a thermal balloon catheter with electrodes 43, as more fully described below. The application of such heat may be employed to facilitate the release of the drug from the coating, to facilitate penetration of the drug into the tissue, or to facilitate the therapeutic action of the drug.

A procedure for preparing a drug delivery balloon with a hydrogel coating and an experiment of drug delivery for the above embodiments are presented in the following examples.

### Examples

### Example 1

A hydrogel coating on an angioplasty balloon may be formed as follows. The surface of the balloon (polyethylene) of an angiographic catheter is prepared by wiping down with clean cloth. The balloon has an O.D. (outer diameter) of about 3.5 mm (inflated). The balloon is coated with a solution of 4,4' diphenylmethane diisocyanate (MDI) in methylethylketone for 30 minutes. After drying in an air oven at 85° C for 30 minutes, the balloon is dipped in a 1.7% solution of poly(acrylic acid) homopolymer having a molecular weight of about 3,000,000 in dimethylformamide (DMF) and tertiarybutyl alcohol. After drying at about 85° C for 30 minutes, a smooth coating is obtained. The balloon is oven dried for 8 hours at 50° C. One function of the drying steps is to remove solvent from the coating. The surface of the balloon becomes instantly lubricous upon exposure to water. The polyisocyanate solution is at a concentration of about .5 to 10% by weight. The polyacrylic acid is at a concentration of about .1 to 10% by weight. The poly(carboxylic acid) to polyisocyanate molar ratio is generally about 1:1. The formation of the hydrogel is further described in US Patent No. 5091205 (USSN 297,331).

A solution of heparin salt may be applied to the coating. The solution is 10,000 units heparin sodium injection (Fisher Scientific, Pittsburgh, PA) USP Grade (1000 units/ml which is then added to 650cc distilled water) and may be applied by dipping for, e.g., about 1 minute at room temperature. The heparin does not form a complex with the hydrogel solution and is freely released in response to compression of the polymer.

After a catheter is prepared for use as discussed above, the catheter may be introduced into the patient using the Seldinger technique and expanded at a desired location to compress the hydrogel and deliver the heparin solution.

### Example 2

Delivery of a drug from a hydrogel coating on a balloon was investigated in the following experiment. Tritium-labeled Pebac was absorbed into a 3.5 mm Slider® (balloon catheter from Boston Scientific Corporation) balloon coated with about a 40 micron thick (in the swelled state) coating as described in Example 1. The coating was dried and the radioactivity was counted. The balloon was then wetted with saline to swell the coating area. The balloon was inflated over a period of about one minute to about 405.300 kPa (about 4 atmospheres) and held at this pressure for about 10 minutes in a thrombus created in an AV shunt from a baboon. The balloon was withdrawn and the amount of the drug in the thrombus was counted with a radiation counter. The experiment was performed with two different balloons using two different concentrations of Pebac, one balloon with 1-2 mg Pebac, and one balloon with 4mg Pebac. Both balloons delivered about 50% of the Pebac into the thrombus.

### Other Embodiments

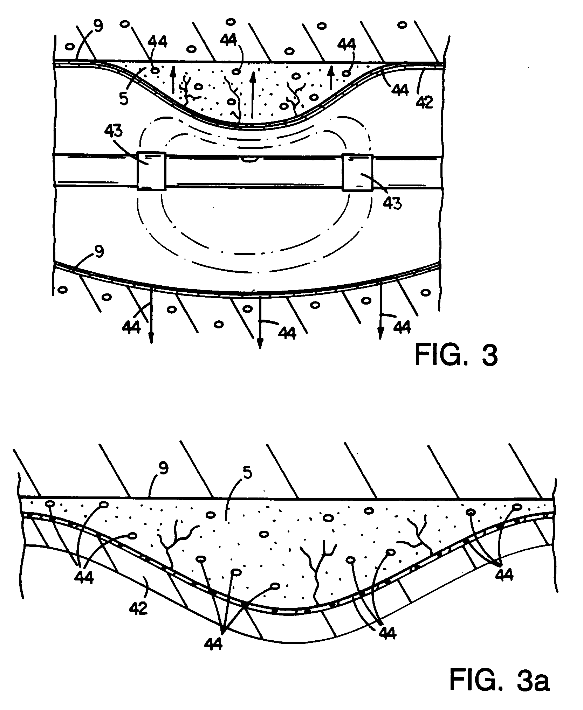

Referring to Fig. 3, in another embodiment, the drug 44 is held within a polymer coating applied to the exterior of a thermal balloon, central wall portions 42 of which are shown in Fig. 3. The balloon is positioned in the lumen in the region to be treated and inflated such that the polymer coating is in contact with the tissue as shown in Figs. 3-3a. Heating of the balloon 42 melts the polymer and releases the drug 44 in a gentle, even, low-energy manner into the affected tissue. Suitable polymers include, but are not limited to, albumin, and collagen, e.g., gelatin, such as gel foams, which typically melt between about 40-60° C or polyvinylpyrrolidone (PVP), which dissolves rapidly when heated. The thermal balloon typically operates between 40-80° C. A suitable heated balloon system for this embodiment or that of Fig. 2a is discussed in Lennox et al., "Heated Balloon Catheters and the Like," U.S. Patent No. 4,955,377. Inflating liquid is heated as a result of I²R losses by radiofrequency current flowing in the inflation fluid, e.g., saline, between the electrodes 43 (see Figs. 2a and 3), the liquid in turn heating the balloon wall. In the alternative, drugs which melt and which could be applied either with a meltable or non-meltable polymer binder might be used.

An advantage to the meltable coatings is that the polymer may be cross-linked, (e.g., by physical or chemical cross-linking) after application of the drug 44 to the balloon to inhibit release of the drug 44 as the balloon 42 is introduced through the body lumen to the area of treatment. The polymer itself typically does not melt off the balloon, but rather softens in a manner permitting release. However, in embodiments where the polymer is bioabsorbable, e.g., polycaprolactone, polyorthoesters, polylactic acids, and polyglycolic acids, some or even all of the polymer may dissolve off of the balloon.

The balloon may also be coated with a polymer incorporating a drug and inflated to press against the wall of the body lumen, where the polymer is selected to separate from the balloon and coat the wall of the lumen, in response to such pressure with or without the application of heat from the balloon. After application of the polymer, the balloon can be deflated and removed. In this embodiment, the polymer may be a blood soluble polymer such as albumin, collagen or the like, incorporating a drug such as heparin. The polymer produces a smooth coating on the wall of the lumen and releases the drug to the tissue over time as the polymer dissolves. Other soluble polymers are meltable and bioabsorbable polymers discussed above.

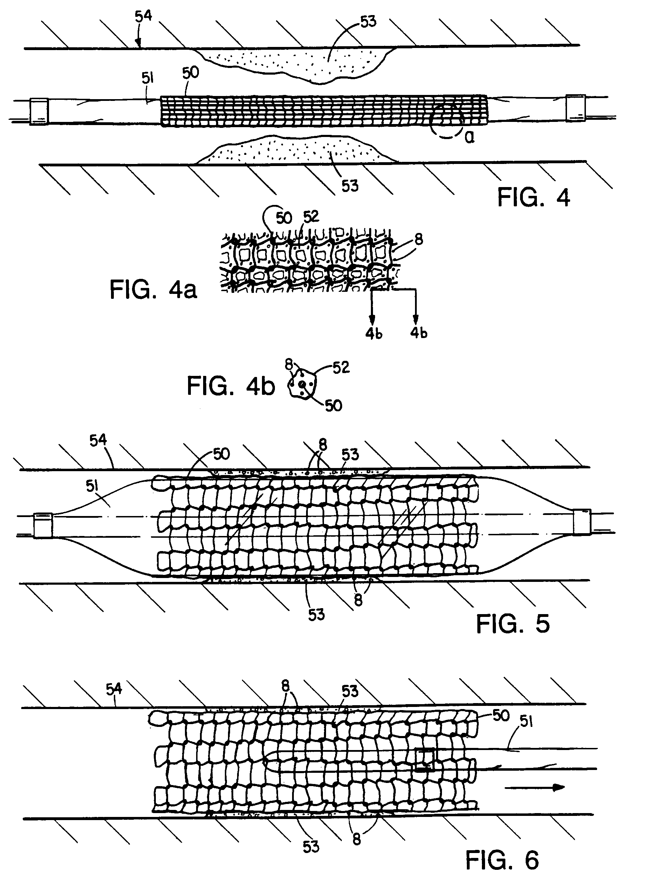

In another embodiment (see Figures 4-6) an endoprosthesis (stent) is used in combination with a balloon catheter drug delivery system. An endoprosthesis 50 is placed over the balloon catheter 51, and then coated with a noncomplexed hydrogel coating 52. The drug 8, shown as circles, in aqueous solution is then absorbed into the hydrogel coating 52. The balloon 51 and hydrogel and drug coated endoprosthesis 50 are slid until they reach the region of the occlusion 53 in the vessel 54. This is shown in Fig. 4. An enlargement of the drug and hydrogel polymer coated endoprosthesis 50 is shown in Figs. 4a and 4b (thickness of coating 52 is greatly exaggerated). After the balloon 51 and hydrogel and drug coated endoprosthesis 50 have been positioned inside the vessel 54, the endoprosthesis 50 is radially expanded by the admission of pressure to the balloon 51 and compressed against the vessel wall 54 with the result that occlusion 53 is compressed, and the vessel wall 54 surrounding it undergoes a radial expansion. The pressure from inflating the balloon squeezes the hydrogel 52, freely releasing the drug 8 into the tissue. The endoprosthesis 50 is held in position in the expanded state as shown in Fig. 5. The pressure is then released from the balloon and the catheter is withdrawn from the vessel. Figure 6 shows the drug 8 inside the compressed thrombus with the endoprosthesis expanded and left in position, with the balloon catheter being withdrawn from the lumen. It will be understood that only the endoprosthesis may include the hydrogel polymer coating. In the embodiments employing a hydrogel-coated stent, the hydrogel and drug are selected such that an initial high dosage of drug is delivered to adjacent tissue upon initial compression of the polymer and thereafter a slow, sustained time-release of drug remaining in the hydrogel polymer occurs. Preferred hydrogel-drug combinations are those that employ a binding of the drug, such as electrostatic binding, e.g., by using a polyacrylic acid hydrogel in combination with an ammonium cation and heparin. In this case, the coating continues to release drug after expansion of the stent and removal of the balloon catheter. The stent may be a balloon-expansible stent as described above or a self-expanding stent, e.g., of the type formed with superelastic materials such as Nitinol.

Any of the embodiments discussed herein can be used with a protective sheath as described in Figs. 2-2a. In addition, a heated balloon catheter may be used in all combinations of the embodiments above to enhance and control the rate of drug-solution delivery into tissue. Other compressible sponge-like polymers, e.g., non hydrogels which release drug solutions in response to pressure, might be used as described with respect to the embodiment of Fig. 1 et seq.

Still other embodiments are within the claims.

## Claims

1. A catheter (1) for delivering drug to tissue at a desired location of the wall of a body lumen (2), comprising:
a catheter (1) constructed for insertion in a body lumen (2) having a catheter shaft (3) and an expandable portion (4) mounted on said catheter shaft (3), said expandable portion (4) being expandable to a controlled pressure to fill the cross-section of the body lumen (2) and press against the wall of said body lumen (2),
at least a portion of the exterior surface of the expandable portion (4) being defined by a coating (6) incorporating an aqueous solution of a preselected drug (8) to be delivered to said tissue,
said coating (6) having the characteristic of releasing said aqueous drug solution (8) in response to compression of said coating (6) against the wall of the lumen (2) when said expandable portion (4) is expanded,
characterised in that said coating comprises a tenaciously adhered swellable hydrogel polymer, said aqueous solution of said preselected drug being incorporated within said hydrogel polymer.

2. The catheter of claim 1 wherein said catheter (1) is a dilatation catheter adapted for insertion in a blood vessel (2), and said expandable portion (4) is an inflatable dilatation balloon adapted for inflation at pressures in the range for effecting widening of a stenosed blood vessel.

3. The catheter of claim 2 wherein said pressure is in the pressure range of about 101.325 to 2026.500 kPa (about 1 to 20 atmospheres).

4. The catheter of claim 1 or 2 wherein said hydrogel polymer and drug are effective to release about 20% or more of said drug during inflation in said pressure range.

5. The catheter of claim 1 or 2 wherein said compression is effective to deliver said drug over a duration of about 10 minutes or less.

6. The catheter of claim 1 or 2 wherein said hydrogel polymer coating is about 10 to 50 microns thick in the swelled, uncompressed state.

7. The catheter of claims 1 or 2 wherein said hydrogel polymer is selected from the group consisting of polycarboxylic acids, cellulosic polymers, gelatin, polyvinylpyrrolidone, maleic anhydride polymers, polyamides, polyvinyl alcohols, and polyethylene oxides.

8. The catheter of claim 7 wherein said hydrogel polymer is polyacrylic acid.

9. The catheter of claim 1 or 2 wherein said drug is an anti-thhrombogenic drug selected from the group consisting of heparin, Pebac, enoxaprin, aspirin and hirudin.

10. The catheter of claim 1 or 2 wherein said drug is an anti-proliferative drug selected from the group consisting of monoclonal antibodies, capable of blocking smooth muscle cell proliferation, angiopeptin and enoxaprin.

11. The catheter of claim 1 or 2 wherein said expandable portion is adapted for application of heat to said polymer material to control the rate of administration.

12. The catheter of claim 1 or 2 wherein said catheter (1) further comprises a sheath member (7), extendable over said balloon (4) to inhibit release of said drug (8) into body fluids during placement of said catheter.

13. The catheter of claim 2 wherein said balloon catheter is a perfusion catheter having an expandable balloon (4).

14. The catheter of any one of claims 1 or 2 wherein said expandable portion includes a stent (50), mountable in said blood vessel by expansion thereof.

15. The catheter of claim 14 where said drug is bound in said hydrogel polymer for slow time release of said drug after said compression of said hydrogel polymer by said expansion.

16. The catheter from claim 15 where said hydrogel polymer is a polyacrylic acid including an ammonium cation and said drug is heparin.

17. The catheter of claim 14 wherein said stent (50) is expandable by a balloon (51).

18. The catheter of claim 17 wherein said stent (50) and balloon (51) both include said swellable hydrogel coating incorporating said drug.

19. The catheter of claim 1 wherein said catheter (1) is a balloon catheter for delivering drug to tissue at a desired location of the wall of a blood vessel (2), comprising:
a catheter (1) constructed for insertion in a blood vessel (2) having a catheter shaft (3) and an expandable dilation balloon (4) mounted on said catheter shaft (3), said expandable balloon (4) being expandable by an expansion controller to engage said tissue at a controlled pressure in the range of about 101.325 to 2026.500 kPa (about 1 to 20 atmospheres) to fill the cross-section of the blood vessel (2) and press against the wall of said blood vessel (2),
at least a portion of the exterior surface of the expandable balloon (4) being defined by said coating (6) of a tenaciously adhered swellable hydrogel polymer having a thickness in the range of about 10 to 50 microns in the swelled state.

20. The catheter of claim 19 wherein said hydrogel polymer is selected from the group consisting of polycarboxylic acids, cellulosic polymers, gelatin, polyvinylpyrrolidone, maleic anhydride polymers, polyamides, polyvinyl alcohols, and polyethylene oxides.

21. The catheter of claim 19 wherein said hydrogel polymer is polyacrylic acid.

22. The catheter of claim 17 or 19 wherein said drug is an anti-thrombogenic drug selected from the group consisting of heparin, Pebac, enoxaprin, aspirin and hirudin.

23. The catheter of claim 17 or 19 wherein said drug is an anti-proliferative drug selected from the group consisting of monoclonal antibodies, capable of blocking smooth muscle cell proliferation, angiopeptin and enoxaprin.

24. The catheter of claim 19 wherein said catheter (1) further comprises a sheath member (7), extendable over said balloon (4) to inhibit release of said drug (8) into body fluids during placement of said catheter.

25. A method for the manufacture of a catheter for delivering drug to tissue at a desired location of the wall of a body lumen (2), the catheter (1) being constructed for insertion in a body lumen (2) and having a catheter shaft (3) and an expandable portion (4) mounted on said catheter shaft (3), said expandable portion (4) being expandable to a controlled pressure to fill the cross-section of the body lumen (2) and press against the wall of said body lumen, characterised by coating at least a portion of the exterior surface of the expandable portion (4) with a tenaciously adhering swellable hydrogel polymer and incorporating said drug (8) to be delivered to said tissue into said hydrogel polymer by introduction thereto as an aqueous solution wherein said hydrogel polymer coating (6) has the characteristic of releasing said aqueous drug solution (8) in response to compression of said coating (6) against the wall of the lumen (2) when said expandable portion (4) is expanded.

## Patentansprüche

1. Katheter (1) zum Transportieren eines Medikaments zu Gewebe an einem gewünschten Ort der Wand eines Körperlumens (2), umfassend:
einen Katheter (1), der zum Einfügen in ein Körperlumen (2) konstruiert ist und einen Katheterschaft (3) und einen auf dem genannten Katheterschaft montierten ausdehnungsfähigen Abschnitt (4) aufweist, wobei der genannte ausdehnungsfähige Abschnitt (4) auf einen geregelten Druck ausdehnbar ist, um den Querschnitt des Körperlumens (2) zu füllen und gegen die Wand des genannten Körperlumens (2) zu drücken,
wobei wenigstens ein Abschnitt der Außenfläche des ausdehnungsfähigen Abschnitts (4) von einer Beschichtung (6) definiert wird, die eine wäßrige Lösung eines vorgewählten Medikaments (8) beinhaltet, das zu dem genannten Gewebe transportiert werden soll,
wobei die genannte Beschichtung (6) die Eigenschaft besitzt, daß sie die genannte wäßrige Medikamentenlösung (8) als Reaktion auf den Druck der genannten Beschichtung (6) gegen die Wand des Lumens (2) freigibt, wenn der genannte ausdehnungsfähige Abschnitt (4) ausgedehnt wird,
dadurch gekennzeichnet, daß die genannte Beschichtung ein zäh anhaftendes, quellfähiges Hydrogelpolymer umfaßt, wobei die genannte wäßrige Lösung des genannten vorgewählten Medikaments in dem genannten Hydrogelpolymer enthalten ist.

2. Katheter nach Anspruch 1, wobei der genannte Katheter (1) ein Dilatationskatheter ist, der zum Einführen in ein Blutgefäß (2) ausgestattet ist, und wobei der genannte ausdehnungsfähige Abschnitt (4) ein füllbarer Dilatationsballon ist, der zum Befüllen mit Drücken in einem Bereich ausgestaltet ist, der eine Erweiterung eines verengten Blutgefäßes bewirkt.

3. Katheter nach Anspruch 2, wobei der genannte Druck im Druckbereich von ca. 101,325 bis 2026,500 kPa (ca. 1 bis 20 atm) liegt.

4. Katheter nach Anspruch 1 oder 2, bei dem das genannte Hydrogelpolymer und das Medikament etwa 20% oder mehr des genannten Medikaments bei der Befüllung in dem genannten Druckbereich freigibt.

5. Katheter nach Anspruch 1 oder 2, bei dem die genannte Kompression bewirkt, daß das genannte Medikament für eine Dauer von etwa 10 Minuten oder weniger zugeführt wird.

6. Katheter nach Anspruch 1 oder 2, bei dem die genannte Hydrogelpolymerbeschichtung im gequollenen, unkomprimierten Zustand eine Dicke von etwa 10 bis 50 Mikron hat.

7. Katheter nach Anspruch 1 oder 2, bei dem das genannte Hydrogelpolymer ausgewählt wird aus der Gruppe bestehend aus Polycarbonsäuren, Cellulosepolymeren, Gelatine, Polyvinylpyrrolidon, Maleinsäureanhydrid-Polymeren, Polyamiden, Polyvinylalkoholen und Polyethylenoxiden.

8. Katheter nach Anspruch 7, wobei das genannte Hydrogelpolymer Polyacrylsäure ist.

9. Katheter nach Anspruch 1 oder 2, bei dem das genannte Medikament ein Anti-Thrombose-Medikament ist, ausgewählt aus der Gruppe bestehend aus Heparin, Pebac, Enoxaprin, Aspirin und Hirudin.

10. Katheter nach Anspruch 1 oder 2, bei dem das genannte Medikament ein Anti-Proliferationsmedikament ist, ausgewählt aus der Gruppe bestehend aus monoklonalen Antikörpern, die eine Proliferation glatter Muskelzellen blockieren können, Angiopeptin und Enoxaprin.

11. Katheter nach Anspruch 1 oder 2, bei dem der genannte ausdehnungsfähige Abschnitt für die Beaufschlagung des genannten Polymermaterials mit Wärme ausgestaltet ist, um die Verabreichungsrate zu kontrollieren.

12. Katheter nach Anspruch 1 oder 2, bei dem der genannte Katheter (1) ferner ein Hüllenelement (7) umfaßt, das über den genannten Ballon (4) ausdehnbar ist, um die Freigabe des genannten Medikamentes (8) in Körperfluide während der Plazierung des genannten Katheters zu hemmen.

13. Katheter nach Anspruch 2, bei dem der genannte Ballonkatheter ein Perfusionskatheter mit einem ausdehnungsfähigen Ballon (4) ist.

14. Katheter nach Anspruch 1 oder 2, bei dem der genannte ausdehnungsfähige Abschnitt einen Stent (50) aufweist, der in dem genannten Blutgefäß durch dessen Ausdehnung befestigt werden kann.

15. Katheter nach Anspruch 14, bei dem das genannte Medikament in dem genannten Hydrogelpolymer für eine langsame Freisetzung des genannten Medikaments nach der genannten Kompression des genannten Hydrogelpolymers durch die genannte Ausdehnung gebunden ist.

16. Katheter nach Anspruch 15, bei dem das genannte Hydrogelpolymer eine Polyacrylsäure mit einem Ammoniumkation und das genannte Medikament Heparin ist.

17. Katheter nach Anspruch 14, bei dem der genannte Stent (50) durch einen Ballon (51) ausgedehnt werden kann.

18. Katheter nach Anspruch 17, bei dem der genannte Stent (50) und der genannte Ballon (51) die genannte, das genannte Medikament beinhaltende quellfähige Hydrogelbeschichtung aufweist.

19. Katheter nach Anspruch 1, wobei der genannte Katheter (1) ein Ballonkatheter zum Transportieren eines Medikamentes zu Gewebe an einem gewünschten Ort der Wand eines Blutgefäßes (2) ist, umfassend:
einen Katheter (1), der zum Einfügen in ein Blutgefäß (2) konstruiert ist und einen Katheterschaft (3) und einen an dem genannten Katheterschaft (3) montierten ausdehnungsfähigen Dilatationsballon (4) aufweist, wobei der genannte ausdehnungsfähige Ballon (4) durch einen Ausdehnungsregler ausgedehnt werden kann, um mit einem geregelten Druck von ca. 101,325 bis 2026,500 kPa (ca. 1 bis 20 atm) in das genannte Gewebe einzugreifen, um den Querschnitt des Blutgefäßes (2) zu füllen und gegen die Wand des genannten Blutgefäßes (2) zu drücken,
wobei wenigstens ein Abschnitt der Außenfläche des ausdehnungsfähigen Ballons (4) von der genannten Beschichtung (6) eines zäh anhaftenden, quellfähigen Hydrogelpolymers mit einer Dicke im Bereich von etwa 10 bis 50 Mikron im gequollenen Zustand definiert wird.

20. Katheter nach Anspruch 19, bei dem das genannte Hydrogelpolymer ausgewählt wird aus der Gruppe bestehend aus Polycarbonsäuren, Cellulosepolymeren, Gelatine, Polyvinylpyrrolidon, Maleinsäureanhydrid-Polymeren, Polyamiden, Polyvinylalkoholen und Polyethylenoxiden.

21. Katheter nach Anspruch 19, bei dem das genannte Hydrogelpolymer Polyacrylsäure ist.

22. Katheter nach Anspruch 17 oder 19, bei dem das genannte Medikament ein Anti-Thrombose-Medikament ist, ausgewählt aus der Gruppe bestehend aus Heparin, Pebac, Enoxaprin, Aspirin und Hirudin.

23. Katheter nach Anspruch 17 oder 19, bei dem das genannte Medikament ein Anti-Proliferationsmedikament ist, ausgewählt aus der Gruppe bestehend aus monoklonalen Antikörpern, die eine Proliferation glatter Muskelzellen blockieren können, Angiopeptin und Enoxaprin.

24. Katheter nach Anspruch 19, bei dem der Katheter (1) ferner ein Hüllenelement (7) umfaßt, das über den genannten Ballon (4) ausdehnbar ist, um die Freigabe des genannten Medikamentes (8) in Körperfluide während der Plazierung des genannten Katheters zu hemmen.

25. Verfahren zur Herstellung eines Katheters für den Transport eine Medikaments zu Gewebe an einem gewünschten Ort der Wand eines Körperlumens (2), wobei der Katheter (1) zum Einfügen in ein Körperlumen (2) konstruiert ist und einen Katheterschaft (3) und einen auf dem genannten Katheterschaft montierten ausdehnungsfähigen Abschnitt (4) aufweist, wobei der genannte ausdehnungsfähige Abschnitt (4) auf einen geregelten Druck ausdehnbar ist, um den Querschnitt des Körperlumens (2) zu füllen und gegen die Wand des genannten Körperlumens zu drücken, gekennzeichnet durch Beschichten von wenigstens einem Abschnitt der Außenfläche des ausdehnungsfähigen Abschnitts (4) mit einem zäh anhaftenden, quellfähigen Hydrogelpolymer, und Einführen des genannten, zu dem genannten Gewebe zu transportierenden Medikamentes (8) in das genannte Hydrogelpolymer durch Einleitung in dieses als wäßrige Lösung, wobei die genannte Hydrogelpolymerbeschichtung (6) die Eigenschaft besitzt, daß sie die genannte wäßrige Medikamentenlösung (8) als Reaktion auf den Druck der genannten Beschichtung (6) gegen die Wand des Lumens (2) freigibt, wenn der genannte ausdehnungsfähige Abschnitt (4) Gausgedehnt wird.

## Revendications

1. Cathéter(1) pour fournir une drogue à un tissu à un emplacement voulu de la paroi d'une lumière corporelle (2), comprenant :
un cathéter (1) construit pour être inséré dans une lumière corporelle (2), comportant une tige de cathéter (3) et une partie expansible (4) montée sur ladite tige de cathéter (3), ladite partie expansible (4) étant expansible à une pression contrôlée pour remplir la section transversale de la lumière corporelle (2) et appuyer contre la paroi de ladite lumière corporelle (2),
au moins une partie de la surface extérieure de la partie expansible (4) étant définie par un revêtement (6) incorporant une solution aqueuse d'une drogue présélectionnée (8) devant être fournie audit tissu,
ledit revêtement (6) ayant la caractéristique de libérer ladite solution de drogue aqueuse (8) en réponse à la compression dudit revêtement (6) contre la paroi de la lumière (2) lorsque ladite partie expansible (4) est dilatée,
caractérisé en ce que ledit revêtement comprend un polymère d'hydrogel gonflable à adhérence tenace, ladite solution aqueuse de ladite drogue présélectionnée étant incorporée dans ledit polymère d'hydrogel.

2. Le cathéter de la revendication 1, dans lequel ledit cathéter (1) est un cathéter à dilatation adapté pour l'introduction dans un vaisseau sanguin (2), et ladite partie expansible (4) est un ballon de dilatation gonflable adapté pour le gonflage à des pressions dans la gamme permettant l'élargissement d'un vaisseau sanguin sténosé.

3. Le cathéter de la revendication 2, dans lequel ladite pression est dans la gamme de pression d'environ 101,325 à 2026,500 kPa (environ 1 à 20 atmosphères).

4. Le cathéter de la revendication 1 ou 2, dans lequel le polymère d'hydrogel et la drogue ont pour effet de libérer environ 20% ou plus de ladite drogue pendant le gonflage dans ladite gamme de pression.

5. Le cathéter de la revendication 1 ou 2, dans lequel ladite compression a pour effet de fournir ladite drogue pendant une durée d'environ 10 minutes ou moins.

6. Le cathéter de la revendication 1 ou 2, dans lequel ledit revêtement de polymère d'hydrogel a une épaisseur d'environ 10 à 50 microns à l'état gonflé, non comprimé.

7. Le cathéter des revendications 1 ou 2, dans lequel ledit polymère d'hydrogel est sélectionné dans le groupe comprenant les acides polycarboxyliques, les polymères cellulosiques, la gélatine, le polyvinylpyrrolidone, les polymères d'anhydride malique, les polyamides, les alcools polyvinyliques, et les oxydes de polyéthylène.

8. Le cathéter de la revendication 7, dans lequel ledit polymère d'hydrogel est l'acide polyacrylique.

9. Le cathéter de la revendication 1 ou 2, dans lequel ladite drogue est une drogue anti-thrombogénique choisie dans le groupe comprenant l'héparine, le Pebac, l'enoxaprine, l'aspirine et l'hirudine.

10. Le cathéter de la revendication 1 ou 2, dans lequel ladite drogue est une drogue anti-prolifère choisie dans le groupe comprenant des anticorps monoclonaux, capable de bloquer la prolifération des cellules musculaires lisses, l'angiopeptine, et l'enoxaprine.

11. Le cathéter de la revendication 1 ou 2, dans lequel ladite partie expansible est adaptée pour l'application de chaleur audit matériau polymère pour contrôler le débit d'administration.

12. Le cathéter de la revendication 1 ou 2, dans lequel ledit cathéter (1) comprend en outre un élément de gaine (7), extensible par-dessus ledit ballon (4) pour inhiber la libération de ladite drogue (8) dans les fluides corporels pendant la mise en place dudit cathéter.

13. Le cathéter de la revendication 2, dans lequel ledit cathéter à ballon est un cathéter de perfusion ayant un ballon expansible (4).

14. Le cathéter de l'une quelconque des revendications 1 ou 2, dans lequel ladite partie expansible comporte un moulage (50) montable dans ledit vaisseau sanguin par son expansion.

15. Le cathéter de la revendication 14, dans lequel ladite drogue est liée dans ledit polymère d'hydrogel pour assurer la libération temporisée de ladite drogue après ladite compression dudit polymère d'hydrogel par ladite expansion.

16. Le cathéter de la revendication 15, où ledit polymère d'hydrogel est un acide polyacrylique comportant un, cation d'ammonium et où ladite drogue est l'héparine.

17. Le cathéter de la revendication 14, dans lequel ledit moulage (50) est expansible par un ballon (51).

18. Le cathéter de la revendication 17, dans lequel ledit moulage (50) et ledit ballon (51) comportent tous deux ledit revêtement d'hydrogel gonflable incorporant ladite drogue.

19. Le cathéter de la revendication 1, dans lequel ledit cathéter (1) est un cathéter à ballon pour fournir une drogue à un tissu à un emplacement voulu de la paroi d'un vaisseau sanguin (2), comprenant :
un cathéter (1) construit pour être introduit dans un vaisseau sanguin (2), ayant une tige de cathéter (3) et un ballon de dilatation expansible (4) monté sur ladite tige de cathéter (3), ledit ballon expansible (4) étant expansible par un contrôleur d'expansion pour engager ledit tissu à une pression contrôlée dans la gamme d'environ 101,325 à 2026,500 kPa (environ 1 à 20 atmosphères) pour remplir la section transversale du vaisseau sanguin (2) et presser contre la paroi dudit vaisseau sanguin (2),
au moins une partie de la surface extérieure du ballon expansible (4) étant définie par ledit revêtement (6) de polymère d'hydrogel gonflable à adhérence tenace ayant une épaisseur dans la gamme d'environ 10 à 50 microns à l'état gonflé.

20. Le cathéter de la revendication 19, dans lequel ledit polymère d'hydrogel est choisi dans le groupe comprenant les acides polycarboxyliques, les polymères cellulosiques, la gélatine, le polyvinylpyrridone, les polymères d'anhydride malique, les polyamides, les alcools polyvinyliques et les oxydes de polyéthylènes.

21. Le cathéter de la revendication, 19 dans lequel ledit polymère d'hydrogel est l'acide polyacrylique.

22. Le cathéter de la revendication 17 ou 19, dans lequel ladite drogue est une drogue anti-thrombogénique choisie dans le groupe comprenant l'héparine, le Pebac, l'enoxaprine, l'aspirine et l'hirudine.

23. Le cathéter de la revendication 17 ou 19, dans lequel ladite drogue est une drogue anti-prolyfère choisie dans le groupe comprenant des anticorps monoclonaux, capable de bloquer la prolifération des cellules musculaires lisses, l'angiopeptine et l'enoxaprine.

24. Le cathéter de la revendication 19, dans lequel ledit cathéter (1) comprend en outre un élément de gaine (7), expansible par-dessus ledit ballon (4) pour inhiber la libération de ladite drogue (8) dans lesdits fluides corporels pendant la mise en place dudit cathéter.

25. Procédé de fabrication d'un cathéter pour fournir une drogue au tissu à un emplacement voulu de la paroi d'une lumière corporelle (2), le cathéter (1) étant construit pour être introduit dans une lumière corporelle (2) et ayant une tige de cathéter (3) et une partie expansible (4) montée sur ladite tige de cathéter (3), ladite partie expansible (4) étant expansible jusqu'à une pression contrôlée pour remplir la section transversale de la lumière corporelle (2) et presser contre la paroi de ladite lumière corporelle, caractérisé par le revêtement d'au moins une partie de la surface extérieure de la partie expansible (4) avec un polymère d'hydrogel gonflable à adhérence tenace et l'incorporation de ladite drogue (8) devant être fournie audit tissu dans ledit polymère d'hydrogel par introduction dans celui-ci sous forme de solution aqueuse, où ledit revêtement de polymère d'hydrogel (6) a la caractéristique de libérer ladite solution aqueuse de drogue (8) en réponse à la compression dudit revêtement (6) contre la paroi de la lumière (2) lorsque ladite partie expansible (4) est dilatée.
